# FASCICULE DE BREVET EUROPEEN

(11) **EP 1 018 009 B1**
(45) Date de publication et mention de la délivrance du brevet: **23.04.2003**
(21) Numéro de dépôt: 98943995.5
(22) Date de dépôt: 16.09.1998
(51) Int. Cl.: G01N 33/542, G01N 33/58

(54) **METHODE HOMOGENE POUR LA DETECTION ET/OU LA DETERMINATION DE L'ACTIVITE PHOSPHORYLANTE D'UN MATERIEL BIOLOGIQUE**
HOMOGENE NACHWEISVERFAHREN ZUR BESTIMMUNG DER PHOSPHORYLIERUNGSAKTIVITÄTEN VON BIOLOGISCHEM MATERIAL
HOMOGENEOUS METHOD FOR DETECTING AND/OR DETERMINING PHOSPHORYLATING ACTIVITY IN A BIOLOGICAL MATERIAL

(30) Priorité: 19.09.1997 FR 9711721
(43) Date de publication de la demande: 12.07.2000
(73) Titulaire: CIS BIO INTERNATIONAL, 91400 Saclay (FR)
(72) Inventeur: MATHIS, Gérard, F-30200 Bagnols sur Cèze (FR); TRINQUET, Eric, F-30150 Pont Saint Esprit (FR); PREAUDAT, Marc, F-30330 Connaux (FR)
(74) Mandataire: Gillard, Marie-Louise
(86) Numéro de dépôt international: FR9801976
(87) Numéro de publication internationale: WO99015896

(56) Documents cités:
- EP-A- 0 180 492
- WO-A-93/05049
- WO-A-96/00901
- WO-A-96/42016
- WO-A-98/02571
- WO-A-98/09169
- US-A- 5 279 943
- US-A- 5 439 797
- GADELLA T W J JR ET AL: "Oligomerization of epidermal growth factor receptors on A431 cells studied by time-resolved fluorescence imaging microscopy: A stereochemical model for tyrosine kinase receptor activation." JOURNAL OF CELL BIOLOGY 129 (6). 1995. 1543-1558. ISSN: 0021-9525, XP002065454
- BROUDY V C ET AL: "Analysis of c-kit receptor dimerization by fluorescence resonance energy transfer." BLOOD 91 (3). 1998. 898-906. ISSN: 0006-4971, XP002065455
- HORROCKS W D JR ET AL: "MEASUREMENT OF DISTANCE BETWEEN FLUORESCENT AMINO-ACID RESIDUES AND METAL ION BINDING SITES QUANTITATION OF ENERGY TRANSFER BETWEEN TRYPTOPHAN AND TERBIUM III OR EUROPIUM III IN THERMO LYSIN EC-3.4.24.4." BIOCHEM BIOPHYS RES COMMUN 100 (1). 1981. 111-117. CODEN: BBRCA9 ISSN: 0006-291X, XP002065456

## Description

L'invention concerne une nouvelle méthode homogène pour la détection et/ou la détermination de l'activité de phosphorylation (ou activité phosphorylante) d'un matériel biologique à l'égard d'un substrat contenant de la tyrosine et/ou de la sérine et/ou de la thréonine, ainsi qu'un kit pour la mise en oeuvre de cette méthode.

La phosphorylation de molécules biologiques telles que des peptides ou des protéines par des kinases est un mécanisme biologique majeur de régulation du métabolisme cellulaire.

La plupart des enzymes possédant une activité de phosphorylation ont un Km (constante de Michaelis) très élevé (généralement compris entre 10⁻³ et 10⁻⁵ M) et un très faible rendement de conversion (entre 5% et 0,001% des sites actifs du substrat sont phosphorylés).

Dans ces conditions, la détection de la phosphorylation d'un substrat n'est possible que si les sites actifs sont présents en large excès pendant la réaction. Ce large excès en sites actifs peut être obtenu soit en utilisant des concentrations élevées en substrat (si celui-ci a peu de sites actifs), soit en choisissant un substrat possédant de nombreux sites de phosphorylation.

Les mécanismes de phosphorylation ont jusqu'à maintenant été généralement étudiés par des méthodes de détection hétérogènes radioactives ou enzymatiques.

Dans ce type de méthodes, la détection de la phosphorylation du substrat fixé sur une phase solide se fait soit par la mesure de l'incorporation de ³²P dans le substrat enzymatique, soit par l'utilisation d'un anticorps marqué (traceur isotopique, enzymatique ou fluorescent) dirigé contre le site de phosphorylation.

Ce type d'essai permet la fixation d'une grande quantité de substrat sur la phase solide et donc la détection de la phosphorylation même lorsque le substrat ne possède qu'un faible nombre de sites actifs, mais présente néanmoins des inconvénients majeurs, à savoir :
- l'utilisation fréquente de marqueurs isotopiques,
- la nécessité de processus de séparation entre les différentes étapes de l'essai pour éliminer les réactifs en excès, et
- la nécessité de maîtriser les processus de « capture » du substrat (comme par exemple lorsqu'on utilise une plaque comportant de l'avidine avec un substrat biotine).

Dans le cas d'une méthode homogène, il est souvent nécessaire que la concentration du substrat soit élevée pour générer suffisamment de substrat phosphorylé à détecter. Il devient alors difficile de capturer la totalité du substrat car cela nécessiterait une quantité importante de réactif, ce qui, si le réactif est fluorescent, présente l'inconvénient de générer un bruit de fond spécifique élevé.

On a maintenant trouvé qu'il était possible de détecter la phosphorylation d'un substrat à l'aide d'une méthode homogène dans laquelle on utilise une molécule porteuse luminescente à laquelle sont couplés de manière covalente une pluralité de substrats. Après la réaction enzymatique de phosphorylation, la quantité de substrat phosphorylé est révélée par la mesure du signal émis par la molécule porteuse luminescente et généré par transfert d'énergie d'un récepteur spécifique du substrat phosphorylé marqué par une molécule luminescente.

Cette méthode est particulièrement utile pour mesurer ta phosphorylation de molécules d'intérêt biologique, telles que par exemple des peptides, des potypeptides, des protéines ou des nucléotides, dans des processus naturels ou pathologique, ou lors de procédés de synthèse comme par exemple la synthèse d'acides nucléiques ou de protéines.

Dans un premier aspect, l'invention a donc pour objet une méthode homogène pour la détection et/ou la détermination de l'activité phosphorylante d'un matériel biologique à l'égard d'un substrat contenant de la tyrosine et/ou de la sérine et/ou de la thréonine, caractérisée en ce que ledit matériel biologique est mis en contact avec une pluralité de peptides ou de polypeptides contenant de la tyrosine et/ou de la sérine et/ou de la thréonine, identiques ou différents, liés de manière covalente à une molécule porteuse, en présence d'une source de phosphate non radiomarqué et des récepteurs spécifiques desdits peptides ou polypeptides phosphorylés, et en ce que la détection et/ou la détermination de l'activité phosphorylante est effectuée par mesure d'un signal d'émission, ledit signal d'émission résultant d'une intéraction entre ladite molécule porteuse constituée par une molécule luminescente ou une molécule non luminescente liée à au moins un marqueur luminescent ou un modulateur du signal d'émission et lesdits récepteurs spécifiques liés à au moins un marqueur luminescent ou un modulateur du signal d'émission.

Par « marqueur luminescent », on entend une molécule luminescente utilisée pour détecter l'interaction entre la molécule porteuse et le récepteur spécifique.

Par « modulateur du signal d'émission », on entend une molécule qui, lorsqu'elle est présente à proximité d'une molécule luminescente, modifie les caractéristiques du signal d'émission de celle-ci.

Selon les molécules utilisées respectivement comme molécule porteuse et comme récepteur spécifique et selon le mécanisme de leur interaction, un même composé luminescent peut jouer le rôle de marqueur luminescent ou de modulateur du signal d'émission.

Ledit modulateur peut être une molécule luminescente, par exemple une molécule luminescente donneur ou accepteur, ou une molécule non luminescente, par exemple un atome de nombre atomique élevé ou une molécule contenant un tel atome comme décrit par exemple dans la demande EP 0 232 348, ou encore des composés marqueurs de spin.

La molécule porteuse peut être :
- soit une molécule luminescente ayant un poids moléculaire élevé, de l'ordre de plusieurs dizaines de kdaltons, comme par exemple une molécule fluorescente telle que l'allophycocyanine ou la C phycocyanine ;
- soit une molécule non luminescente, comme par exemple la thyroglobuline, liée à au moins un marqueur luminescent ou à au moins un modulateur du signal d'émission ;
- soit un solide dispersé luminescent ayant une surface suffisante pour fixer une pluralité de peptides ou polypeptides substrats ;
- soit un solide dispersé non luminescent ayant une surface suffisante pour fixer une pluralité de peptides ou polypeptides substrats, lié à au moins un marqueur luminescent ou à au moins un modulateur du signal d'émission.

La molécule porteuse peut donc être soit une molécule luminescente accepteur, soit une molécule non luminescente liée à au moins un marqueur luminescent ou à au moins un modulateur du signal d'émission.

Dans la suite de la description, on emploiera sans distinction les termes « molécule » ou « composé» pour qualifier les marqueurs luminescents ou les modulateurs liés à la molécule porteuse ou au récepteur spécifique.

Dans un aspect avantageux, la molécule porteuse est soit une molécule fluorescente accepteur, soit une molécule fluorescente donneur, soit une molécule non fluorescente liée à au moins un composé fluorescent accepteur, ou à au moins un composé fluorescent donneur.

Avantageusement, le marqueur luminescent ou le modulateur du signal d'émission lié à chacun des récepteurs spécifiques du ou des peptide(s) ou polypeptide(s) phosphorylé(s) peut être une molécule fluorescente donneur ou accepteur.

Dans un aspect préféré, la détection et/ou la détermination de l'activité de phosphorylation est effectuée par mesure du signal d'émission résultant du transfert d'énergie non radiatif entre la molécule porteuse et les marqueurs luminescents ou les modulateurs du signal d'émission liés aux récepteurs spécifiques des peptides ou polypeptides phosphorylés.

Ainsi, le signal d'émission lumineuse permettant la détection et/ou la détermination de l'activité phosphorylante recherchée peut être généré soit par transfert d'énergie non radiatif des marqueurs luminescents ou des modulateurs du signal d'émission liés aux récepteurs spécifiques à la molécule porteuse, soit inversement par transfert d'énergie non radiatif des marqueurs luminescents ou des modulateurs du signal d'émission de la molécule porteuse aux marqueurs luminescents liés aux récepteurs spécifiques.

Par "transfert d'énergie entre la molécule porteuse et les molécules luminescentes marqueurs ou les modulateurs du signal d'émission liés aux récepteurs spécifiques des peptides ou polypeptides phosphorylés", on entend donc les 2 types de mécanismes ci-dessus.

Le transfert d'énergie non radiatif, dont le principe est notamment décrit dans G.Mathis et al., Clin. Chem., 1993, 39, 1953-1959 est réalisé lorsque les conditions suivantes sont remplies :
- d'une part, le composé accepteur possède un spectre d'absorption qui recouvre au moins partiellement le spectre d'émission du donneur et présente une absorbance molaire élevée dans cette zone de recouvrement, et un spectre d'émission dans une gamme de longueur d'ondes où le donneur présente une émission intrinsèque faible ;
- d'autre part, l'accepteur et le donneur se situent à proximité l'un de l'autre.

La quantité de peptides ou de polypeptides liés de manière covalente à la molécule luminescente porteuse peut être d'environ 2 à 1000 par molécule luminescente porteuse.

Les récepteurs spécifiques des peptides ou polypeptides phosphorylés peuvent être par exemple choisi parmi les anticorps monoclonaux ou polyclonaux.

Dans un aspect préféré, le composé luminescent lié au récepteur spécifique du ou des peptide(s) ou polypeptide(s) phosphorylé(s) ou à la molécule porteuse, en tant que marqueur luminescent ou de modulateur du signal d'émission selon le mécanisme de l'interaction entre ladite molécule porteuse et ledit récepteur spécifique, est un chelate, un cryptate ou un complexe macrocyclique d'ion terre rare.

Dans la suite de la description, les termes "chelate", et "cryptate" ainsi que la nomenclature des macrocycles et polycycles utilisables sont tels que définis par J.M.Lehn dans Struct. Bonding (Berlin), 16, 1 1973 et dans Acc. Chem. Res.. 11, 49 (1979).

Ledit composé fluorescent donneur est de préférence un cryptate de terre rare choisi de préférence parmi les cryptates de terbium, d'europium, de samarium, de dysprosium ou de néodymium.

Selon un aspect préféré, ledit cryptate de terre rare est constitué d'au moins un sel de terre rare complexé par un composé macropolycyclique de formule 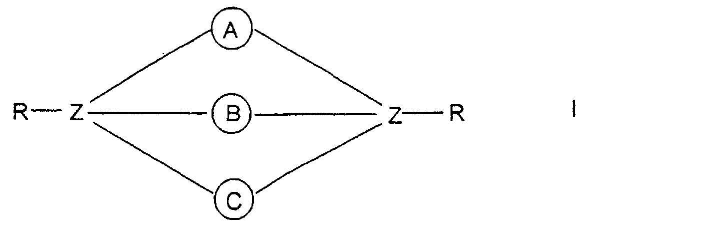 dans laquelle Z est un atome ayant 3 ou 4 valences, R est rien ou représente l'hydrogène, le groupe hydroxy, un groupe amino ou un radical hydrocarboné, les radicaux bivalents 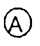 , 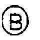 et 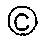 , sont indépendamment l'un de l'autre des chaînes hydrocarbonées qui contiennent éventuellement un ou plusieurs hétéroatomes et sont éventuellement interrompues par un hétéromacrocycle, au moins l'un des radicaux 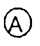 , 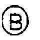 et 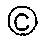 comportant de plus au moins un motif moléculaire ou étant essentiellement constitué par un motif moléculaire, ledit motif moléculaire possédant une énergie de triplet supérieure à celle du niveau émissif de l'ion de terre rare complexé.

De préférence, il s'agit d'un cryptate de formule (I) ci-dessus dans laquelle le motif moléculaire est choisi parmi la phénanthroline, l'anthracène, le benzène, le naphtalène, les bi- et ter-phényle, l'azobenzène, l'azopyridine, la pyridine, les bipyridines, les bisquinoléines et les composés de formules ci-après :

- C₂H₄ - X₁ - C₆H₄ - X₂ - C₂H₄ -

- C₂H₄ - X₁ - CH₂ - C₆H₄ - CH₂ - X₂ - C₂H₄ -

X₁ et X₂ pouvant être identiques ou différents désignent l'oxygène, l'azote ou le soufre, 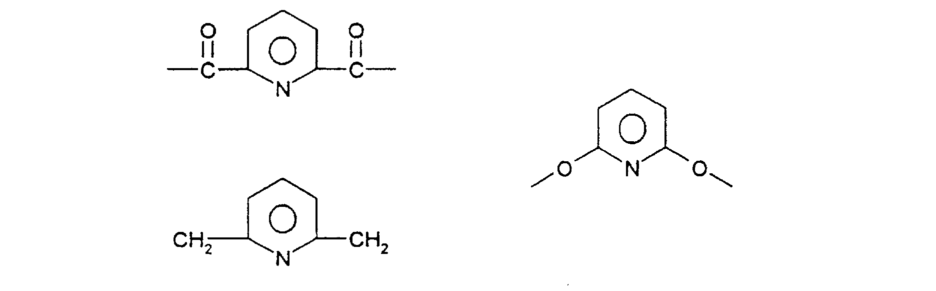 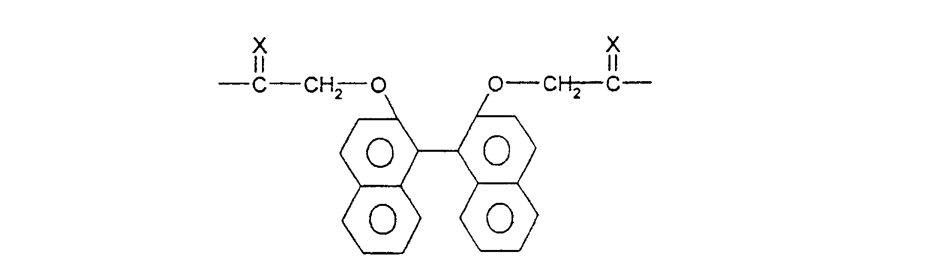 X étant l'oxygène ou l'hydrogène.

Dans un aspect avantageux, le composé fluorescent est un cryptate de terre rare constitué de l'ion terbium ou europium complexé par l'un des composés macrocycliques ci-après :
(22)phénanthroline ; (22)phénanthroline amide ; (22)anthracène ; (22)anthracène amide ; (22)bi-isoquinoléine ; (22)biphényl-bis-pyridine ; (22)bipyridine ; (22)bi-pyridine amide ; les macropolycycles tris-bipyridine, tris-phénanthroline, phénanthroline-bis-bipyridine, bi-isoquinoléine-bis-bipyridine, bis-bipyridine diphénylbipyridine.

Un composé fluorescent particulièrement avantageux est le cryptate d'europium Eu tris bipyridine.

De tels composés sont par exemple décrits dans le brevet EP 180 492.

On peut également utiliser des composés macrocycliques complexant des ions de terre rare dans lesquels le motif moléculaire est choisi parmi les bipyrazines, les bipyrimidines et les hétérocycles azotés comportant des groupes N-oxydes.

Des composés macrocycliques à unités bipyrazines sont décrits dans F. Bodar-Houillon et al., New J. Chem., 1996, 20, 1041-1045.

Des composés macrocycliques à unités bipyrimidines sont décrits dans J. M. Lehn et al., Helv. Chim. Acta, 1992, 75, 1221.

Des composés macrocycliques comprenant des hétérocycles azotés comportant des groupes N-oxydes sont décrits dans J.M. Lehn et al., Helv. Chim. Acta, 1991, 74, 572 et dans le brevet EP 0 601 113.

Le cryptate de terre rare utilisé comme composé fluorescent donneur peut également être constitué d'au moins un sel de terre rare complexé par un composé macropolycyclique répondant à l'une des formules Il ou III ci-après : 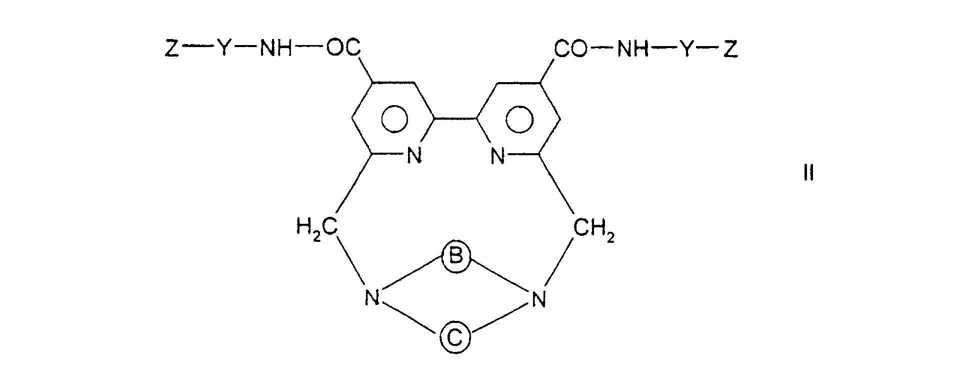 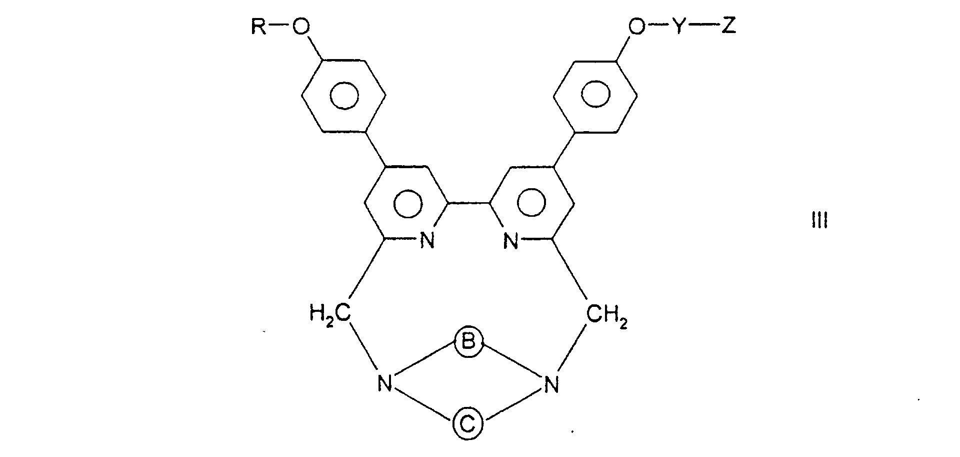 dans lesquels :
- le cycle de formule 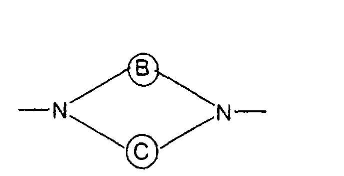 est l'un des cycles suivants : 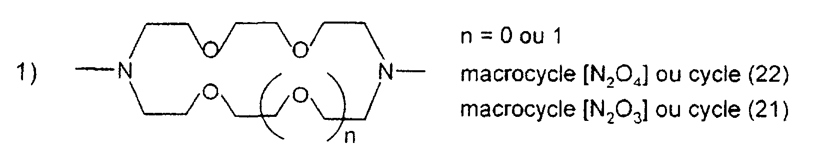 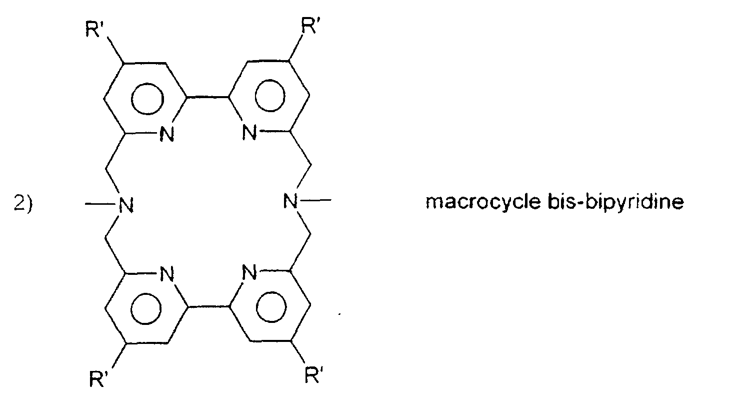
- Y est un groupe ou un bras d'espacement qui est constitué par un radical organique bivalent, choisi parmi les groupes alkylène linéaires ou ramifiés en C₁ à C₂₀ contenant éventuellement une ou plusieurs doubles liaisons ou triples liaisons et/ou étant éventuellement interrompus par un ou plusieurs hétéroatomes tels que l'oxygène, l'azote, le soufre ou le phosphore, parmi les groupes cycloalkylène en C₅ à C₈ ou parmi les groupes arylène en C₆ à C₁₄, lesdits groupes alkylène, cycloalkylène ou arylène étant éventuellement substitués par des groupes alkyle, aryle ou sulfonate ;
- Z est un groupe fonctionnel susceptible de se lier de façon covalente avec une substance biologique ;
- R est un groupe méthyle ou représente le groupe -Y-Z ;
- R' est l'hydrogène ou un groupe -COOR" dans lequel R" est un groupe alkyle en C₁ à C₁₀ et représente de préférence le groupe méthyle, éthyle ou tertiobutyle ou bien R' est un groupe -CO-NH-Y-Z.

De tels composés sont décrits par exemple dans le brevet EP 321 353.

Dans la méthode selon l'invention, ledit composé fluorescent peut être lié au récepteur spécifique ou à la molécule porteuse soit directement, soit par l'intermédiaire d'un bras d'espacement.

Ce bras d'espacement est par exemple constitué par un radical organique bivalent, choisi parmi les groupes alkylène linéaires ou ramifiés en C₁-C₂₀. contenant éventuellement une ou plusieurs doubles liaisons et/ou éventuellement interrompus par un ou plusieurs hétéroatomes, tels que l'oxygène, l'azote, le soufre ou le phosphore; les groupes carbamoyle et carboxamido ; les groupes cycloalkylène en C₅-C₈ et les groupes arylène en C₆-C₁₄, lesdits groupes alkylène, cycloalkylène ou arylène étant éventuellement substitués par des groupes alkyle. aryle ou sulfonate.

Dans un aspect préféré, on utilisera en tant que composé fluorescent donneur lié au récepteur spécifique un cryptate d'europium et, en tant que molécule porteuse ou composé fluorescent accepteur lié à la molécule porteuse, l'allophycocyanine, l'allophycocyanine B, un dérivé d'allophycocyanine chimiquement modifié, la C phycocyanine, la R phycocyanine et les cyanines.

Dans un autre aspect avantageux, on utilisera en tant que composé fluorescent donneur lié au récepteur spécifique un cryptate de terbium et en tant que molécule porteuse ou composé fluorescent accepteur lié à la molécule porteuse, les rhodamines, la thionine, la R phycocyanine, la phycoerythrocyanine, la C phycoerythrine, la B phycoerythrine, la R phycoerythrine et les cyanines.

Des composés fluorescents utilisables également comme composés accepteurs sont les complexes phycobiliprotéine-peptide de liaison décrits dans la demande WO96142016.

Selon un autre de ses aspects, l'invention concerne également un kit pour la détection et/ou la détermination de l'activité phosphorylante d'un matériel biologique à l'égard d'un substrat contenant de la tyrosine et/ou de la sérine et/ou de la thréonine, caractérisé en ce qu'il contient au moins une molécule porteuse à laquelle sont fixés de manière covalente une pluralité de peptides ou de polypeptides contenant de la tyrosine et/ou de la sérine et/ou de la thréonine, identiques ou différents, et au moins un récepteur spécifique desdits peptides ou polypeptides phosphorylés, ledit récepteur étant lié à au moins un marqueur luminescent ou un modulateur du signal d'émission.

La molécule porteuse est telle que définie plus haut, c'est-à-dire qu'elle peut être luminescente de manière intrinsèque ou par liaison à au moins un marqueur luminescent ou un modulateur du signal d'émission.

Avantageusement la molécule porteuse et le marqueur luminescent ou le modulateur du signal d'émission lié au récepteur spécifique de ce kit sont des composés fluorescents.

Dans un aspect préféré, le composé luminescent (marqueur luminescent
ou modulateur du signal d'émission) lié au récepteur spécifique et la molécule porteuse sont respectivement des composés fluorescents donneur et accepteur.

Le composé luminescent lié au récepteur spécifique dans le kit selon l'invention peut être le cryptate d'europium Eu trisbipyridine ou le cryptate de terbium Tb trisbipyridine.

Avantageusement, le kit selon l'invention contient en outre un milieu tampon approprié, une source de phosphate non radiomarqué et des instructions pour la mise en oeuvre de la méthode de détection et/ou de la détermination de l'activité phosphorylante d'un matériel biologique décrite plus haut.

L'invention est illustrée par l'exemple ci-après.

### Exemple 1 : Détection de la phosphorylation du peptide SRC

Le peptide SRC est un substrat de la tyrosine kinase du récepteur du facteur de croissance épidermique, également dénommé EGF pour "Epidermal Growth Factor". C'est un peptide de 11 acides aminés contenant un seul motif tyrosine et présentant la structure suivante :

[H]-Leu-Ile-Glu-Asp-Ala-Glu-Tyr-Ala-Ala-Gly-[OH]

Les abréviations utilisées ci-après sont les suivantes :
DTT = dithiotreitrol
EuTBP = cryptate d'europium Eu trisbipyridine diamine
BSA = sérum albumine bovine
IgG = immunoglobuline G
MHS = maléimidohexanoyl-N-hydroxy-succinimidester
SPDP = N-succinimidyl 3(2-pyridyldithio)propionate
Sulfo-SMCC = sulfosuccinimidyl 4-N-maléimidométhyl)cyclohexane.

### 1) Conjugaison de la molécule porteuse luminescente avec le peptide substrat

On utilise un dérivé d'allophycocyanine chimiquement modifié (XL₆₆₅, Cis bio international) dont le poids moléculaire élevé autorise son marquage par de nombreux peptides possédant chacun un site de phosphorylation.

### a) Activation de XL₆₆₅ par SPDP

A 6 mg d' XL₆₆₅ à 3,45 mg/ml dans un tampon phosphate 100 mM pH 7,0, on ajoute une solution de 80 mM de SPDP dans l'éthanol absolu dans une proportion de 60 moles d'activateur par mole de XL₆₆₅.

Après 30 minutes d'activation à température ambiante on ajoute une solution de 200 mM de DTT dans un tampon phosphate 100 mM pH 7,0 dans une proportion de 5 moles de réducteur par mole d'activateur.

Après 15 minutes à température ambiante, les produits réactionnels indésirables sont éliminés par chromatographie d'exclusion-diffusion sur colonne gel G25 superfine dans un tampon phosphate 100 mM pH 6,5, EDTA 5 mM.

Le produit est conservé à 4°C avant couplage.

### b) Activation du peptide par le MHS

A 4 mg de peptide (2,6 pmoles), on ajoute une solution 220 mM de MHS dans l'acétonitrile dans une proportion de 4 moles d'activateur par mole de peptide.

Après 30 minutes à température ambiante, les produits réactionnels indésirables sont éliminés par chromatographie d'exclusion-diffusion sur colonne gel Superdex 30 ® (PHARMACIA) dans un tampon phosphate 100 mM pH 7,0.

### c) Couplage peptide-maléimide / XL₆₆₅-SH

De façon similaire à celle décrite plus haut, on fait réagir les fonctions maléimides avec les fonctions thiols fixées sur la XL₆₆₅ dans des proportions molaires de 100 peptide par XL₆₆₅.

Après 18 heures d incubation à 40°C et blocage des groupements thiols (éventuellement restés libres) par N-éthylmaléimide, le peptide non couplé est éliminé par chromatographie d'exclusion-diffusion sur colonne TSK 3000 SW (MERCK) en tampon phosphate 100 mM pH 7,0.

On obtient un conjugué comportant entre 20 et 40 peptides par molécule XL₆₆₅.

### 2) Préparation du conjugué anticorps anti-phosphotvrosine/cryptate d'Europium Eu trisbipyridine

### a) Activation des IgG PY20 par le SPDP

5 mg d'IgG PY20 (Transduction Laboratories) à raison de 10 mg/ml dans un tampon phosphate 100 mM, pH 7,0 sont activés par l'ajout d'une solution de SPDP (Pierce, USA) à raison de 6,4 mM dans du dioxane dans un rapport molaire de 7,5 SPDP par IgG PY20.

Après 35 min d'activation à température ambiante, l'IgG pyridine-2-thione est purifiée sur colonne G25 superfine dans un tampon phosphate 100 mM, EDTA 5mM, pH 6,5.

Les protéines sont concentrées et les groupes 2-pyridyl disulfides sont réduits par une solution de DTT (Sigma, USA) ayant une concentration finale de 19 mM pendant 15 min à température ambiante. Le DTT et la pyridine-2-thione sont éliminés par purification sur colonne G25 superfine en tampon phosphate 100 mM, EDTA 5 mM, pH 6,5. La concentration en IgG-SH est déterminée à 280 nm avec un ε₂₈₀ₙₘ de 210 000 M⁻¹ cm⁻¹.

### b) Préparation des conjugués IgG PY20-EuTBP

A 5 mg (5 10⁻⁶ moles) de Eu TBP (cryptate d'Europium Eu trisbipyridine diamine préparé comme décrit dans le brevet EP 321 353, exemples 3 et 4) est ajoutée une solution à 25 mM de sulfo-SMCC, en tampon phosphate 20 mM, diméthylformamide 10 % (v/v pH 7.0) dans une proportion de 2,5 moles d'activateur par mole de Eu TBP.

Après 45 min d'activation à température ambiante, le milieu réactionnel est filtré à 0,8 µm afin d'éliminer le précipité éventuellement formé. Les produits réactionnels indésirables (suifo-SMCC, N-hydroxysuccinimide, acide (N-maléimidométhyl)carboxylique) sont éliminés par chromatographie echangeuse d'ions sur colonne Mono Q (Pharmacia, Suède) en tampon phosphate 20 mM diméthylformamide 10 % (v/v), pH 7,0 sous choc de NaCl. La concentration en Eu TBP maléimide est déterminée à 307 nm avec un ε₃₀₇ nm de 25 000 M⁻¹cm⁻¹ ainsi que le rapport A₃₀₇ₙₘ/A₂₈₀ₙₘ.

De façon similaire à celle décrite plus haut, on fait réagir les fonctions maléimides avec les fonctions thiols fixés sur l'anticorps, dans des proportions molaires variant de 10 à 30 Eu TBP maléimide par IgG PY20-SH.

Après 18 heures d'incubation à 4°C et blocage des groupements thiols (éventuellement restés libres) par N-éthytmaléimide, le Eu TBP non couplé est éliminé par dialyse en tampon phosphate 100 mM pH 7,0 à 4°C jusqu'à épuisement (plus de fluorescence dans les bains de dialyse).

Les caractéristiques du conjugué sont déterminées par ses absorptions à 307 nm et à 280 nm en utilisant les valeurs suivantes en tenant compte de l'absorption propre du cryptate déterminée par le rapport A₃₀₇ₙₘ/A₂₈₀ₙₘ.
Eu TBP-maléimide :
ε₃₀₇ₙₘ = 25 000 M⁻¹ cm⁻¹
A₃₀₇ₙₘ/A₂₈₀ₙₘ = déterminée expérimentalement.
IgG PY20-SH :
ε₂₈₀ₙₘ = 210 000 M⁻¹ cm⁻¹

### 3) Phosphorylation

Des cellules A431 (SIGMA) contenant un récepteur de l'EGF sont préactivées 10 min à température ambiante par de l'EGF. Le tampon de phosphocylation est un tampon TRIS/MES 60 mM, pH 7,4 contenant 30 µM d'ATP, 50 mM de Mg⁺⁺ et 10 mM de Mn⁺⁺.

On ajoute successivement dans les puits « essais » d'une microplaque à 96 puits :
- 10 µl de cellules A431 préactivées,
- 10 µl de conjugué XL₆₆₅-peptides SRC
- 30 µl de tampon de phosphorylation.

Dans les puits « blancs » servant de contrôle, on introduit 10 µl de conjugué XL₆₆₅-peptides et 40 µl de tampon de phosphorylation. On incube ensuite 30 min à température ambiante.

### 4) Révélation

On ajoute successivement dans chaque puits de la microplaque :
- 50 µl d'anticorps/anti-phosphotyrosine marqué au cryptate Eu trisbipyridine
- 100 µl de tampon phosphate 0,1 M ; pH 7 ; KF 0,4 M ; BSA 0,1 %.

Après incubation 30 min à température ambiante, la lecture de la fluorescence est effectuée à 620 nm et 665 nm à l'aide d'un fluorimètre à laser prototype, décrit ci-après :
Un laser pulsé à azote (LASER SCIENCE INC., modèle LS1-337ND) est utilisé comme source d'excitation (longueur d'onde à 337,1 nm). La durée des pulsations est spécifiée à 3 nanosecondes et est répétée sous une fréquence de 10 Hertz. Le faisceau passe à travers un filtre (CORNING) afin d'éliminer toute lumière parasite à l'excitation autre que 337 nm.

Après être rentré dans la chambre de mesure, le faisceau est réfléchi par un filtre dichroïque, placé à 45 degrés, qui a la propriété de réfléchir les ultraviolets et de pouvoir transmettre la lumière visible.

Le faisceau réfléchi par le filtre dichroïque est focalisé sur le puits à mesurer d'une microplaque par une lentille en silice fondue. L'émission de fluorescence est collectée selon un angle solide de 20 degrés, collimatée par la même lentille, et passe directement à travers le filtre dichroïque (fluorescence en lumière visible).

Un filtre interférentiel de caractéristiques définies selon la longueur d'onde de fluorescence à détecter, permet de se débarrasser des lumières pouvant parasiter le signal, dont l'intensité est ensuite mesurée par un photomultiplicateur (HAMAMATSU R2949).

Le compteur de photons utilisé est un SR-400 (STANFORD RESEARCH SYSTEMS), dont les opérations et la synchronisation avec le laser sont contrôlées par un ordinateur de type IBM PC-AT via une sortie RS 232. Les pulsations provenant du photomultiplicateur sont enregistrées pendant une fenêtre de temps (tg) et après un délai (t_{d}) déterminés à condition qu'elles soient supérieures à un niveau discriminant sélectionné par le compteur de photons afin d'optimiser le rapport signal/bruit du photomultiplicateur.

Une table X-Y, pilotée par l'IBM PC-AT, permet les différents positionnements de la microplaque de mesure par des moteurs pas à pas, incluant les manoeuvres de chargement, de positionnement sous le faisceau excitant, de lecture automatique en séquentiel des 96 puits, et de sortie.

La fluorescence émise par le conjugué XL₆₆₅-peptides SRC est mesurée à l'aide du fluorimètre prototype équipé d'un filtre à 665 nm de 10 nm de largeur à mi-hauteur, pendant 400 µs et avec un délai de 50 µs.

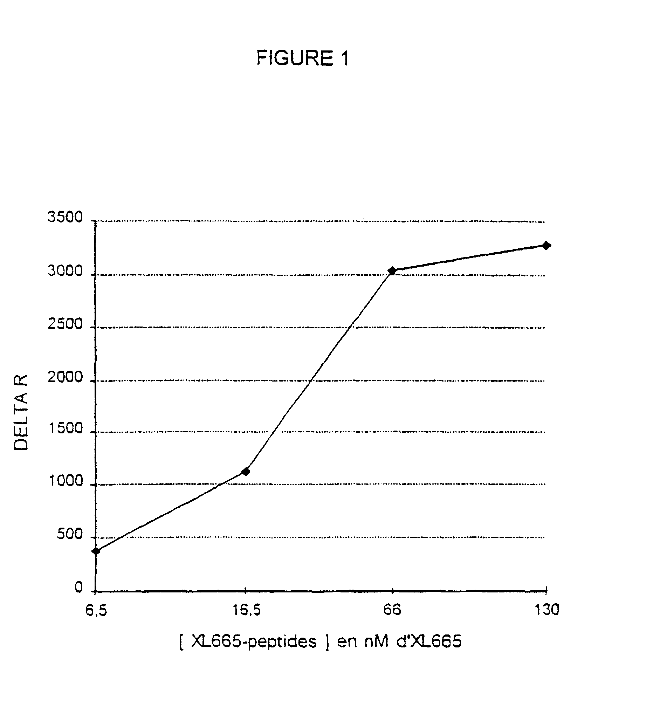

Les résultats sont représentés sur le graphe de la figure 1, dans lequel l'axe des ordonnées donne le taux de phosphorylation et l'axe des abscisses donne la concentration en conjugué XL₆₆₅-peptides SRC.

Le taux de phosphorylation est exprimé par la variable DR = R_{échantillon}-R_{blanc}, R étant le rapport des signaux d'émission à 665 nm et à 620 nm.

La concentration en conjugué XL₆₆₅-peptides SRC est exprimée en nM de XL₆₆₅.

Les résultats montrent que l'augmentation du taux de phosphorylation est corrélée à l'augmentation de la concentration de conjugué XL₆₆₅-peptides SRC.

## Revendications

1. Méthode homogène pour la détection et/ou la détermination de l'activité phosphorylante d'un matériel biologique à l'égard d'un substrat contenant de la tyrosine et/ou de la sérine et/ou de la thréonine, **caractérisée en ce que** ledit matériel biologique est mis en contact avec une pluralité de peptides ou de polypeptides contenant de la tyrosine et/ou de la sérine et/ou de la thréonine, identiques ou différents, liés de manière covalente à une molécule porteuse, en présence d'une source de phosphate non radiomarqué et des récepteurs spécifiques desdits peptides ou polypeptides phosphorylés, et **en ce que** la détection et/ou la détermination de l'activité phosphorylante est effectuée par mesure d'un signal d'émission, ledit signal d'émission résultant d'une intéraction entre ladite molécule porteuse constituée par une molécule luminescente ou une molécule non luminescente liée à au moins un marqueur luminescent ou un modulateur du signal d'émission et lesdits récepteurs spécifiques liés à au moins un marqueur luminescent ou un modulateur du signal d'émission.

2. Méthode selon la revendication 1, **caractérisée en ce que** la détection et/ou la détermination de l'activité phosphorylante est effectuée par mesure du signal d'émission résultant du transfert d'énergie entre les marqueurs luminescents ou les modulateurs du signal d'émission liés aux récepteurs spécifiques des peptides ou polypeptides phosphorylés et la molécule porteuse.

3. Méthode selon l'une des revendications 1 ou 2 **caractérisée en ce que** la molécule porteuse luminescente est une molécule fluorescente ou une molécule non fluorescente liée à au moins un marqueur fluorescent ou à au moins un modulateur du signal d'émission.

4. Méthode selon l'une quelconque des revendications 1 à 3, **caractérisée en ce que** le marqueur luminescent ou le modulateur du signal d'émission lié au récepteur spécifique est un composé fluorescent.

5. Méthode selon l'une quelconque des revendications 1 à 4, **caractérisée en ce que** le récepteur spécifique est lié à un composé fluorescent donneur et la molécule porteuse est une molécule fluorescente accepteur ou est liée à un composé fluorescent accepteur.

6. Méthode selon l'une quelconque des revendications 1 à 5, **caractérisée en ce que** le récepteur spécifique est lié à un composé fluorescent accepteur et la molécule porteuse luminescente est une molécule fluorescente donneur ou est liée à un composé fluorescent donneur.

7. Méthode selon l'une quelconque des revendications 1 à 6, **caractérisée en ce que** la quantité de peptides ou polypeptides liés de manière covalente à la molécule porteuse luminescente est de 2 à 1000.

8. Méthode selon l'une quelconque des revendications 1 à 7, **caractérisée en ce que** le récepteur spécifique est lié à un chélate, un cryptate ou un complexe macrocyclique d'ion terre rare.

9. Méthode selon la revendication 8, **caractérisée en ce que** le récepteur spécifique est lié à un chélate, un cryptate ou un complexe macrocyclique d'europium, de terbium, de dysprosium, de samarium ou de néodymium.

10. Méthode selon la revendication 9, **caractérisée en ce que** le récepteur spécifique est lié à un cryptate de terre rare constitué d'au moins un sel de terre rare complexé par un composé macropolycyclique de formule 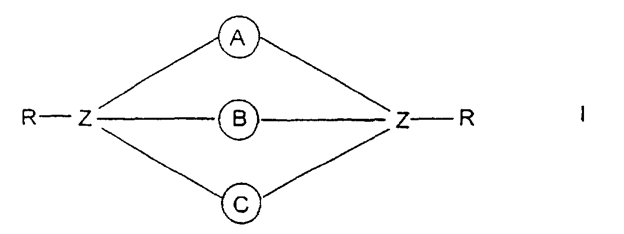 dans laquelle Z est un atome ayant 3 ou 4 valences, R est rien ou représente l'hydrogène, le groupe hydroxy, un groupe amino ou un radical hydrocarboné, les radicaux bivalents 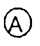 , 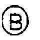 et 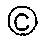 , sont indépendamment l'un de l'autre des chaînes hydrocarbonées qui contiennent éventuellement un ou plusieurs hétéroatomes et sont éventuellement interrompues par un hétéromacrocycle, au moins l'un des radicaux 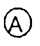 , 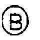 et 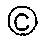 comportant de plus au moins un motif moléculaire ou étant essentiellement constitué par un motif moléculaire, ledit motif moléculaire possédant une énergie de triplet supérieure à celle du niveau émissif de l'ion de terre rare complexé.

11. Méthode selon la revendication 10, **caractérisée en ce que** le composé fluorescent lié au récepteur spécifique est un cryptate de terre rare constitué de l'ion terbium ou europium complexé par l'un des composés macrocycliques ci-après :
(22)phénanthroline; (22)phénanthroline amide; (22)anthracène; (22)anthracène amide ; (22)bi-isoquinoléine; (22)biphényl-bis-pyridine; (22)bipyridine; (22)bi-pyridine amide; les macropolycycles tris-bipyridine, tris-phénanthroline, phénanthroline-bis-bipyridine, bi-isoquinoléine-bis-bipyridine, bis-bipyridine diphénylbipyridine.

12. Méthode selon la revendication 11, **caractérisée en ce que** ledit composé fluorescent est le cryptate d'europium Eu trisbipyridine.

13. Méthode selon l'une quelconque des revendications 1 à 12, **caractérisée en ce qu'**on utilise un cryptate d'europium en tant que composé fluorescent donneur lié au récepteur spécifique et en tant que molécule porteuse ou composé fluorescent accepteur lié à la molécule porteuse, l'allophycocyanine, l'allophycocyanine B. les dérivés d'allophycocyanine chimiquement modifiés, la C phycocyanine. la R phycocyanine et les cyanines.

14. Méthode selon l'une quelconque des revendications 1 à 12, **caractérisée en ce qu'**on utilise un cryptate de terbium en tant que composé fluorescent donneur lié au récepteur spécifique et, en tant que molécule porteuse ou composé fluorescent accepteur lié à la molécule porteuse, les rhodamines, la thionine, la R phycocyanine, la phycoerythrocyanine, la C phycoerythrine, la B phycoerythrine, la R phycoerythrine et les cyanines.

15. Méthode selon l'une des revendications 1 à 14, **caractérisée en ce que** le composé fluorescent donneur lié au récepteur spécifique est le cryptate d'europium Eu trisbipyridine ou le cryptate de terbium Tb trisbipyridine.

16. Méthode selon l'une quelconque des revendications 1 à 15, **caractérisée en ce que** les récepteurs spécifiques sont choisis parmi les anticorps potyclonaux et monoclonaux.

17. Kit pour la détection et/ou la détermination de l'activité phosphorylante d'un matériel biologique à l'égard d'un substrat contenant de la tyrosine et/ou de la sérine et/ou de la thréonine, **caractérisé en ce qu'**il contient au moins une molécule porteuse constituée par une molécule luminescente ou une molécule non luminescente liée à au moins un marqueur luminescent ou un modulateur du signal d'émission à laquelle sont fixés de manière covalente une pluralité de peptides ou de polypeptides contenant de la tyrosine et/ou de la sérine et/ou de la thréonine, identiques ou différents, et au moins un récepteur spécifique desdits peptides ou polypeptides phosphorylés, ledit récepteur étant lié à au moins un marqueur ou un modulateur du signal d'émission.

18. Kit selon la revendication 17, **caractérisé en ce que** la molécule porteuse et la molécule luminescente marqueur du récepteur spécifique sont des molécules fluorescentes.

19. Kit selon la revendication 18, **caractérisé en ce que** le marqueur luminescent ou le modulateur du signal d'émission lié au récepteur spécifique et la molécule porteuse sont respectivement des molécules fluorescentes donneur et accepteur.

20. Kit selon l'une quelconque des revendications 17 à 19, **caractérisé en ce que** le composé luminescent lié au récepteur spécifique est le cryptate d'europium Eu trisbipyridine ou le cryptate de terbium Tb trisbipyridine.

21. Kit selon l'une quelconque des revendications 17 à 20, **caractérisé en ce qu'**il contient également un milieu tampon approprié, une source de phosphate non radiomarqué et des instructions pour la mise en oeuvre de la méthode selon l'une quelconque des revendications 1 à 16.

## Patentansprüche

1. Homogenes Verfahren zum Nachweisen und/oder Bestimmen der Phosphorylierungsaktivität eines biologischen Materials in bezug auf ein Substrat, das Tyrosin und/oder Serin und/oder Threonin enthält, **dadurch gekennzeichnet, daß** man das biologische Material mit mehreren Peptiden oder Polypeptiden, die Tyrosin und/oder Serin und/oder Threonin enthalten, die gleich oder verschieden sind und die kovalent an ein Trägermolekül gebunden sind, in Gegenwart einer nicht radioaktiv markierten Phosphatquelle und von spezifischen Rezeptoren dieser phosphorylierten Peptide oder Polypeptide in Kontakt bringt sowie dadurch, daß man die Phosphorylierungsaktivität dadurch nachweist und/oder bestimmt, daß man ein Emissionssignal mißt, wobei dieses Emissionssignal von einer Wechselwirkung zwischen dem aus einem lumineszierenden Molekül oder einem nicht lumineszierenden Molekül, das an mindestens einen Lumineszenzmarker oder einen Emissionssignalmodulator gebunden ist, bestehenden Trägermolekül und den am mindestens einen Lumineszenzmarker oder einen Emissionssignalmodulator gebundenen spezifischen Rezeptoren herrührt.

2. Verfahren nach Anspruch 1, **dadurch gekennzeichnet, daß** der Nachweis und/oder die Bestimmung der Phosphorylierungsaktivität dadurch durchgeführt wird, daß man das Emissionssignal, das durch den Energietransfer zwischen den Luminiszenzmarkern oder den an die spezifischen Rezeptoren der phosphorylierten Peptide oder Polypeptide gebundenen Emissionssignalmodulatoren und dem Trägermolekül entsteht, mißt.

3. Verfahren nach Anspruch 1 oder 2, **dadurch gekennzeichnet, daß** es sich bei dem lumineszierenden Trägermolekül um ein fluoreszierendes Molekül oder ein nicht fluoreszierendes Molekül, das an mindestens einen Fluoreszenzmarker oder an mindestens einen Emissionssignalmodulator gebunden ist, handelt.

4. Verfahren nach einem der Ansprüche 1 bis 3, **dadurch gekennzeichnet, daß** es sich bei dem Lumineszenzmarker oder dem Emissionssignalmodulator, der an den spezifischen Rezeptor gebunden ist, um eine fluoreszierende Verbindung handelt.

5. Verfahren nach einem der Ansprüche 1 bis 4, **dadurch gekennzeichnet, daß** der spezifische Rezeptor an eine fluoreszierende Donatorverbindung gebunden ist und das Trägermolekül ein fluoreszierendes Akzeptormolekül ist oder an eine fluoreszierende Akzeptorverbindung gebunden ist.

6. Verfahren nach einem der Ansprüche 1 bis 5, **dadurch gekennzeichnet, daß** der spezifische Rezeptor an eine fluoreszierende Akzeptorverbindung gebunden ist und das lumineszierende Trägermolekül ein fluoreszierendes Donatormolekül ist oder an eine fluoreszierende Donatorverbindung gebunden ist.

7. Verfahren nach einem der Ansprüche 1 bis 6, **dadurch gekennzeichnet, daß** die Menge der kovalent an das lumineszierende Trägermolekül gebundenen Peptide oder Polypeptide 2 bis 1000 beträgt.

8. Verfahren nach einem der Ansprüche 1 bis 7, **dadurch gekennzeichnet, daß** der spezifische Rezeptor an ein Chelat, ein Kryptat oder einen makrozyklischen Komplex eines Seltene-Erde-Ions gebunden ist.

9. Verfahren nach Anspruch 8, **dadurch gekennzeichnet, daß** der spezifische Rezeptor an ein Chelat, ein Kryptat oder einen makrozyklischen Komplex des Europiums, Terbiums, Dysprosiums, Samariums oder Neodymiums gebunden ist.

10. Verfahren nach Anspruch 9, **dadurch gekennzeichnet, daß** der spezifische Rezeptor an ein Seltene-Erde-Kryptat gebunden ist, das aus mindestens einem durch eine makropolyzyklische Verbindung der Formel 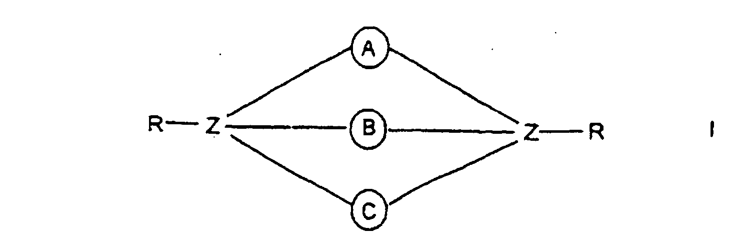 in der Z ein Atom mit 3 oder 4 Valenzen bedeutet, R Null oder Wasserstoff, die Hydroxygruppe, eine Aminogruppe oder einen Kohlenwasserstoffrest bedeutet, die zweiwertigen Reste 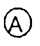 , 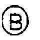 und 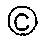 unabhängig voneinander Kohlenhydratketten, die gegebenenfalls ein oder mehrere Heteroatome enthalten und die gegebenenfalls durch einen Heteromakrozyklus unterbrochen sind, bedeuten, wobei mindestens einer der Reste 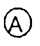 , 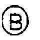 und 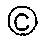 außerdem mindestens ein Molekülmotiv enthält oder im wesentlichen aus einem Molekülmotiv besteht, das über eine Triplett-Energie verfügt, die die Triplett-Energie des Emissionsniveaus des komplexierten Seltene-Erde-Ions übertrifft, komplexierten Seltene-Erde-Salz besteht.

11. Verfahren nach Anspruch 10, **dadurch gekennzeichnet, daß** die an den spezifischen Rezeptor gebundene fluoreszierende Verbindung ein Seltene-Erde-Kryptat ist, das aus dem mit einer der im folgenden genannten makrocyclischen Verbindungen komplexierten Terbium- oder Europiumion besteht:
(22)Phenantrolin, (22)Phenantrolinamid, (22)Anthracen, (22)Anthracenamid, (22)Biisochinolin, (22)Biphenyl-bis-pyridin, (22)Bipyridin, (22)Bipyridinamid, die Makropolyzyklen Tris-bipyridin, Trisphenanthrolin, Phenanthrolin-bis-bipyridin, Biisochinolin-bis-bipyridin, Bisbipyridindiphenylbipyridin.

12. Verfahren nach Anspruch 11, **dadurch gekennzeichnet, daß** es sich bei der fluoreszierenden Verbindung um das Europiumkryptat Eu-tris-bipyridin handelt.

13. Verfahren nach einem der Ansprüche 1 bis 12, **dadurch gekennzeichnet, daß** man ein Europiumkryptat als fluoreszierende Donatorverbindung, die an den spezifischen Rezeptor gebunden ist, und Allophycocyanin, Allophycocyanin B, die chemisch modifizierten Allophycocyaninderivate, Phycocyanin C, Phycocyanin R sowie die Cyanine als Trägermolekül bzw. als fluoreszierende Akzeptorverbindung, die an das Trägermolekül gebunden ist, verwendet.

14. Verfahren nach einem der Ansprüche 1 bis 12, **dadurch gekennzeichnet, daß** man ein Terbiumkryptat als fluoreszierende Donatorverbindung, die an den spezifischen Rezeptor gebunden ist, und die Rhodamine, Thionin, Phycocyanin R, Phycoerythrocyanin, Phycoerythrin C, Phycoerythrin B, Phycoerythrin R sowie die Cyanine als Trägermolekül bzw. als fluoreszierende Akzeptorverbindung, die an das Trägermolekül gebunden ist, verwendet.

15. Verfahren nach einem der Ansprüche 1 bis 14, **dadurch gekennzeichnet, daß** es sich bei der fluoreszierenden Donatorverbindung, die an den spezifischen Rezeptor gebunden ist, um das Europiumkryptat Eu-trisbipyridin oder das Terbiumkryptat Tb-trisbipyridin handelt.

16. Verfahren nach einem der Ansprüche 1 bis 15, **dadurch gekennzeichnet, daß** die spezifischen Rezeptoren aus der Gruppe der polyklonalen und monoklonalen Antikörper stammen.

17. Kit zum Nachweisen und/oder Bestimmen der Phosphorylierungsaktivität eines biologischen Materials in bezug auf ein Substrat, das Tyrosin und/oder Serin und/oder Threonin enthält, **dadurch gekennzeichnet, daß** es mindestens ein aus einem lumineszierenden Molekül oder einem nicht lumineszierenden Molekül, das an mindestens einen Lumineszenzmarker oder einen Emissionssignalmodulator gebunden ist, bestehendes Trägermolekül, wobei an dieses Trägermolekül mehrere Peptide oder Polypeptide, die Tyrosin und/oder Serin und/oder Threonin enthalten und gleich oder verschieden sind, kovalent gebunden sind, sowie mindestens einen spezifischen Rezeptor für diese phosphorylierten Peptide oder Polypeptide enthält, wobei der Rezeptor an mindestens einen Marker oder Emissionssignalmodulator gebunden ist.

18. Kit nach Anspruch 17, **dadurch gekennzeichnet, daß** es sich bei dem Trägermolekül und dem lumineszierenden Molekül als Marker für den spezifischen Rezeptor um fluoreszierende Moleküle handelt.

19. Kit nach Anspruch 18, **dadurch gekennzeichnet, daß** es sich bei dem Lumineszenzmarker oder dem Emissionssignalmodulator, der an den spezifischen Rezeptor und das Trägermolekül gebunden ist, um fluoreszierende Donator- bzw. Akzeptormoleküle handelt.

20. Kit nach einem der Ansprüche 17 bis 19, **dadurch gekennzeichnet, daß** es sich bei der lumineszierenden Verbindung, die an den spezifischen Rezeptor gebunden ist, um das Europiumkryptat Eu-trisbipyridin oder das Terbiumkryptat Tb-trisbipyridin handelt.

21. Kit nach einem der Ansprüche 17 bis 20, **dadurch gekennzeichnet, daß** es auch ein entsprechendes Puffermedium, eine nicht radioaktiv markierte Phosphatquelle sowie eine Gebrauchsanweisung für die Durchführung des Verfahrens nach einem der Ansprüche 1 bis 16 enthält.

## Claims

1. Homogeneous method of detecting and/or determining the phosphorylating activity of a biological material towards a substrate containing tyrosine and/or serine and/or threonine, **characterized in that** said biological material is brought into contact with a plurality of identical or different peptides or polypeptides containing tyrosine and/or serine and/or threonine, covalently bonded to a carrier molecule, in the presence of a source of non-radiolabeled phosphate and specific receptors for said phosphorylated peptides or polypeptides, and **in that** the phosphorylating activity is detected and/or determined by measuring an emission signal, said emission signal resulting from an interaction between said carrier molecule, consisting of a luminescent molecule or a non-luminescent molecule bonded to at least one luminescent marker or at least one emission signal modulator, and said specific receptors bonded to at least one luminescent marker or at least one emission signal modulator.

2. Method according to claim 1, **characterized in that** the phosphorylating activity is detected and/or determined by measuring the emission signal resulting from the energy transfer between the carrier molecule and the luminescent markers or the emission signal modulators bonded to the specific receptors for the phosphorylated peptides or polypeptides.

3. Method according to one of claims 1 or 2, **characterized in that** the luminescent carrier molecule is a fluorescent molecule or a non-fluorescent molecule bonded to at least one fluorescent marker or at least one emission signal modulator.

4. Method according to any one of claims 1 to 3, **characterized in that** the luminescent marker or the emission signal modulator bonded to the specific receptor is a fluorescent compound.

5. Method according to any one of claims 1 to 4, **characterized in that** the specific receptor is bonded to a fluorescent donor compound and the carrier molecule is a fluorescent acceptor molecule or is bonded to a fluorescent acceptor compound.

6. Method according to any one of claims 1 to 5, **characterized in that** the specific receptor is bonded to a fluorescent acceptor compound and the luminescent carrier molecule is a fluorescent donor molecule or is bonded to a fluorescent donor compound.

7. Method according to any one of claims 1 to 6, **characterized in that** the amount of peptides or polypeptides covalently bonded to the luminescent carrier molecule is 2 to 1000.

8. Method according to any one of claims 1 to 7, **characterized in that** the specific receptor is bonded to a chelate, a cryptate or a macrocyclic complex of a rare earth ion.

9. Method according to claim 8, **characterized in that** the specific receptor is bonded to a chelate, a cryptate or a macrocyclic complex of europium, terbium, dysprosium, samarium or neodymium.

10. Method according to claim 9, **characterized in that** the specific receptor is bonded to a rare earth cryptate consisting of at least one rare earth salt complexed by a macropolycyclic compound of the formula 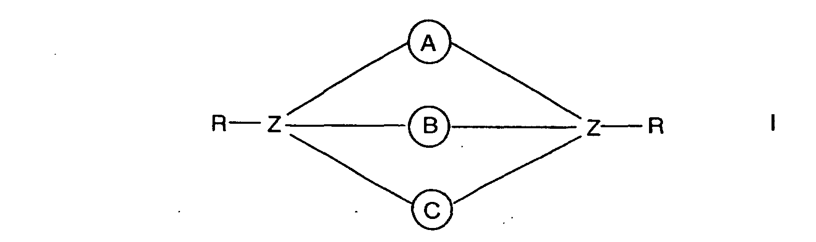 in which Z is a trivalent or tetravalent atom, R is nothing, hydrogen, the hydroxyl group, an amino group or a hydrocarbon radical, and the divalent radicals 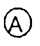 , 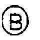 and 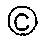 independently of one another are hydrocarbon chains which optionally contain one or more heteroatoms and are optionally interrupted by a heteromacrocycle, at least one of the radicals 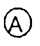 , 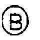 and 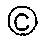 also containing at least one molecular moiety or essentially consisting of a molecular moiety, said molecular moiety possessing a greater triplet energy than that of the emission level of the complexed rare earth ion.

11. Method according to claim 10, **characterized in that** the fluorescent compound bonded to the specific receptor is a rare earth cryptate consisting of the terbium or europium ion complexed by one of the following macrocyclic compounds:
(22)phenanthroline; (22)phenanthroline amide; (22)anthracene; (22)anthracene amide; (22)biisoquinoline; (22)biphenyl-bis-pyridine; (22)bipyridine; (22)bipyridine amide; and tris-bipyridine, tris-phenanthroline, phenanthroline-bisbipyridine, biisoquinoline-bis-bipyridine and bis-bipyridine diphenylbipyridine macropolycycles.

12. Method according to claim 11, **characterized in that** said fluorescent compound is the europium cryptate Eu tris-bipyridine.

13. Method according to any one of claims I to 12, **characterized in that** a europium cryptate is used as a fluorescent donor compound bonded to the specific receptor, and allophycocyanin, allophycocyanin B, a chemically modified allophycocyanin derivative, C phycocyanin, R phycocyanin or a cyanin is used as a carrier molecule or a fluorescent acceptor compound bonded to the carrier molecule.

14. Method according to any one of claims 1 to 12, **characterized in that** a terbium cryptate is used as a fluorescent donor compound bonded to the specific receptor, and a rhodamine, thionine, R phycocyanin, phycoerythrocyanin, C phycoerythrin, B phycoerythrin, R phycoerythrin or a cyanin is used as a carrier molecule or a fluorescent acceptor compound bonded to the carrier molecule.

15. Method according to one of claims 1 to 14, **characterized in that** the fluorescent donor compound bonded to the specific receptor is the europium cryptate Eu tris-bipyridine or the terbium cryptate Tb tris-bipyridine.

16. Method according to any one of claims 1 to 15, **characterized in that** the specific receptors are selected from polyclonal and monoclonal antibodies.

17. Kit for detecting and/or determining the phosphorylating activity of a biological material towards a substrate containing tyrosine and/or serine and/or threonine, **characterized in that** it contains at least one carrier molecule, consisting of a luminescent molecule or a non-luminescent molecule bonded to at least one luminescent marker or at least one emission signal modulator, to which a plurality of identical or different peptides or polypeptides containing tyrosine and/or serine and/or threonine, are covalently fixed, and at least one specific receptor for said phosphorylated peptides or polypeptides, said receptor being bonded to at least one marker or at least one emission signal modulator.

18. Kit according to claim 17, **characterized in that** the carrier molecule and the luminescent marker molecule bonded to the specific receptor are fluorescent molecules.

19. Kit according to claim 18, **characterized in that** the luminescent marker or the emission signal modulator bonded to the specific receptor, and the carrier molecule, are respectively fluorescent donor and acceptor molecules.

20. Kit according to any one of claims 17 to 19, **characterized in that** the luminescent compound bonded to the specific receptor is the europium cryptate Eu tris-bipyridine or the terbium cryptate Tb tris-bipyridine.

21. Kit according to any one of claims 17 to 20, **characterized in that** it also contains an appropriate buffer medium, a source of non-radiolabeled phosphate and instructions for carrying out the method according to any one of claims 1 to 16.
